# EUROPÄISCHE PATENTSCHRIFT

(11) **EP 2 681 905 B1**
(45) Veröffentlichungstag und Bekanntmachung des Hinweises auf die Patenterteilung: **25.04.2018**
(21) Anmeldenummer: 12706788.2
(22) Anmeldetag: 02.03.2012
(51) Int. Cl.: A61B 6/03, G01T 1/24, H01L 27/146, H04N 5/32

(54) **RÖNTGENKAMERA ZUR ORTSAUFGELÖSTEN DETEKTION VON RÖNTGENSTRAHLUNG**
X-RAY CAMERA FOR THE SPATIALLY-RESOLVED DETECTION OF X-RAY
CAMÉRA A RAYONNEMENT X POUR LA DÉTECTION À RÉSOLUTION SPATIALE DE RAYONNEMENT X

(30) Priorität: 04.03.2011 DE 102011013058
(43) Veröffentlichungstag der Anmeldung: 08.01.2014
(73) Patentinhaber: Helmholtz Zentrum München Deutsches Forschungszentrum für Gesundheit und Umwelt (GmbH), 85764 Neuherberg (DE)
(72) Erfinder: THALHAMMER, Stefan, 80639 München (DE); HOFSTETTER, Markus, 85579 Neubiberg (DE); HOWGATE, John, 80335 München (DE); STUTZMANN, Martin, 85435 Erding (DE)
(74) Vertreter: Hertz, Oliver
(86) Internationale Anmeldenummer: PCT/EP2012/000946
(87) Internationale Veröffentlichungsnummer: WO 2012/119741

(56) Entgegenhaltungen:
- EP-A2- 1 744 368
- US-A- 5 742 659
- MALINOWSKI P E ET AL: "Backside-Illuminated GaN-on-Si Schottky Photodiodes for UV Radiation Detection", IEEE ELECTRON DEVICE LETTERS, IEEE SERVICE CENTER, NEW YORK, NY, US, Bd. 30, Nr. 12, 1. Dezember 2009 (2009-12-01), Seiten 1308-1310, XP011284249, ISSN: 0741-3106, DOI: 10.1109/LED.2009.2033722
- YVES NGU ET AL: "Array of Two UV-Wavelength Detector Types", IEEE TRANSACTIONS ON ELECTRON DEVICES, IEEE SERVICE CENTER, PISACATAWAY, NJ, US, Bd. 57, Nr. 6, 1. Juni 2010 (2010-06-01), Seiten 1224-1229, XP011307992, ISSN: 0018-9383
- HOFSTETTER MARKUS ET AL: "Real-time x-ray response of biocompatible solution gate AlGaN/GaN high electron mobility transistor devices", APPLIED PHYSICS LETTERS, AIP, AMERICAN INSTITUTE OF PHYSICS, MELVILLE, NY, US, Bd. 96, Nr. 9, 5. März 2010 (2010-03-05), Seiten 92110-92110, XP012132404, ISSN: 0003-6951, DOI: 10.1063/1.3334682
- DUBOZ JEAN-YVES ET AL: "Anomalous photoresponse of GaN x-ray Schottky detectors", JOURNAL OF APPLIED PHYSICS, AMERICAN INSTITUTE OF PHYSICS, US, vol. 105, no. 11, 9 June 2009 (2009-06-09) , pages 114512-114512, XP012125603, ISSN: 0021-8979, DOI: 10.1063/1.3141818

## Beschreibung

Die Erfindung betrifft eine Röntgenkamera zur ortsaufgelösten Detektion von Röntgenstrahlung. Des weiteren betrifft die Erfindung ein Verfahren zur Aufnahme eines Bildes eines zu Untersuchungsgegenstandes unter Verwendung von Röntgenstrahlung und Anwendungen der Röntgenkamera.

Es ist bekannt, Galliumnitrid (GaN) in Halbleiterdetektoren zur Detektion von Röntgenstrahlung zu verwenden. Beispielsweise wird in US 2010/0069749 A1 ein GaN-Sensor beschrieben, der in Reaktion auf eine Röntgenbestrahlung Lumineszenzlicht emittiert. Das Lumineszenzlicht wird vom Sensor über einen Lichtleiter zu einem Photodetektor geleitet. Diese Technik hat Nachteile, da die Kombination des Sensors dem Lichtleiter einen empfindlichen Aufbau darstellt und da die detektierte Röntgenstrahlung nicht direkt in ein elektrisches Messsignal umgewandelt wird. Der GaN-Sensor gemäß US 2010/0069749 A1 ist aufgrund seines Aufbaus insbesondere für eine Anordnung einer Vielzahl von Detektoren (Array-Anordnung) ungeeignet.

Des weiteren wird von J.-Y. Duboz et al. in "Applied Physics Letters" Band 92, 2008, S. 263501, die Eignung von GaN zur Detektion von Röntgenstrahlung untersucht. Hierzu wurden GaN-Schichten, z. B. mit einer Dicke von 110 µm oder 480 µm, auf Silizium- oder Saphir-Substraten abgeschieden und mit Kontaktelektroden versehen, die mit der GaN-Schicht einen Schottky-Kontakt bildeten. Es wurde jedoch festgestellt, dass eine zuverlässige Detektion auf Röntgenstrahlung mit einer Energie unterhalb von 20 keV beschränkt war. Für praktische Anwendungen eines Strahlungsdetektors, zum Beispiel in der Dosimetrie, ist jedoch eine Empfindlichkeit für Röntgenstrahlung mit einer Energie oberhalb von 20 keV erforderlich.

Schließlich wird von M. Hofstetter et al. in "Applied Physics Letters" Band 96, 2010, S. 092110, ein Strahlungsdetektor für Röntgenstrahlung beschrieben, der einen sogenannten HEMT ("High Electron Mobility Transistor") mit einem GaN-basierten Mehrschichtsystem enthält. Dieser Strahlungsdetektor kann ebenfalls aufgrund seines Mehrschichtaufbaus Nachteile haben.

In der Praxis hat sich gezeigt, dass die bisher beschriebenen Strahlungsdetektoren auf GaN-Basis insbesondere aufgrund ihres komplexen Aufbaus, einer komplizierten Kalibrierung und/oder einer unzureichenden Empfindlichkeit für einen routinemäßigen Einsatz in der Dosimetrie ungeeignet sind. Des weiteren sind bisher keine praktikablen Array-Anordnungen von GaN-basierten Strahlungsdetektoren, zum Beispiel in der Medizintechnik, der Materialprüfung oder der Strahlungsüberwachung beschrieben worden.

Weitere Detektoreinrichtungen zur Erfassung kurzwelliger elektromagnetischer Strahlung sind aus US 5 742 659 A und EP 1 744 368 A2 bekannt.

Die Aufgabe der Erfindung ist es, eine verbesserte Array-Anordnung von Strahlungsdetektoren anzugeben, mit der Nachteile herkömmlicher Techniken überwunden werden. Die Array-Anordnung soll sich insbesondere durch einen vereinfachten Aufbau, einen vereinfachten Betrieb und/oder eine erhöhte Empfindlichkeit im Vergleich zu herkömmlichen Detektoren auszeichnen. Die Aufgabe der Erfindung ist es des Weiteren, Verfahren zur Röntgen-Bildgebung und Anwendungen der Array-Anordnung anzugeben, mit denen Nachteile herkömmlicher Techniken zur Detektion von Röntgenstrahlung überwunden werden.

Diese Aufgaben werden durch eine Röntgenkamera und Verfahren mit den Merkmalen der unabhängigen Ansprüche gelöst. Vorteilhafte Ausführungsformen und Anwendungen der Erfindung ergeben sich aus den abhängigen Ansprüchen.

Gemäß einem ersten allgemeinen Gesichtspunkt der Erfindung wird eine Röntgenkamera zur Detektion von Röntgenstrahlung nach dem beigefügten Anspruch 1, bereitgestellt, die insbesondere eine Vielzahl von Strahlungsdetektoren umfasst, die an einer Halteeinrichtung mit einer vorbestimmten Anordnung positioniert sind. Die Strahlungsdetektoren sind an der Halteeinrichtung entlang einer vorbestimmten Bezugslinie (1D-Kamera, Kamerazeile) oder Bezugsfläche (2D-Kamera) fixiert. Die Halteeinrichtung bildet eine Befestigung der Strahlungsdetektoren. Die Strahlungsdetektoren weisen eine vorbestimmte geometrische Anordnung auf der Halteeinrichtung auf, so dass Messsignale der Strahlungsdetektoren eine geometrische Verteilung von Dosiswerten in einem Strahlungsfeld der Röntgenstrahlung repräsentieren können und aus den Messsignalen der Strahlungsdetektoren ein Bild der Dosiswerte ermittelbar ist. Wenn die Röntgenstrahlung nach einer Wechselwirkung mit einem Untersuchungsgegenstand (z.B. Durchstrahlung oder Wechselwirkung mit einer Oberfläche des Untersuchungsgegenstandes) in charakteristischer Weise durch Eigenschaften des Untersuchungsgegenstandes verändert ist, liefern die Messsignale der Strahlungsdetektoren ein Bild des Untersuchungsgegenstandes, wie z.B. ein Durchstrahlungsbild oder ein Reflektionsbild.

Gemäß der Erfindung umfasst jeder Strahlungsdetektor, d. h. jedes Pixel der Röntgenkamera, ein Trägersubstrat, eine auf dem Trägersubstrat angeordnete GaN-basierte Detektorschicht und mit der Detektorschicht verbundene Kontaktelektroden. Gemäß der Erfindung hat die Detektorschicht eine Dicke, die geringer als 50 µm ist. Die Trägersubstrate der Strahlungsdetektoren können miteinander zu einem einheitlichen Substrat verbunden sein. Die Detektorschichten können, z. B. durch Ätzen, aus einer auf dem Substrat gebildeten einheitlichen GaN-basierten Schicht gebildet sein. Des weiteren ist gemäß der Erfindung vorgesehen, dass die Kontaktelektroden mit der Detektorschicht ohmsche Kontakte bilden.

Die Erfinder haben festgestellt, dass eine einzige, mit ohmschen Kontakten versehene Detektorschicht mit der genannten, im Vergleich zu herkömmlichen Strahlungsdetektoren erheblich verringerten Schichtdicke eine empfindliche, reproduzierbare und nur eine einfache Widerstands- oder Leitfähigkeitsmessung erfordernde Detektion von Röntgenstrahlung ermöglicht. Bei Anlegen einer Spannung an die mit den Kontakten versehene Detektorschicht liefert eine Strommessung einen Widerstands- oder Leitfähigkeitswert. Die Detektorschicht wirkt wie ein Photoleiter, dessen elektrischer Widerstand sich in Reaktion auf Röntgenbestrahlung ändert. An den Kontaktelektroden ist ein Detektorstrom messbar. Die detektierte Röntgenstrahlung wird somit direkt in ein elektrisches Messsignal (Detektorstrom, Widerstands- oder Leitfähigkeitswert) konvertiert. Die Konvertierungsausbeute ist dabei so groß, dass der erfindungsgemäße Strahlungsdetektor miniaturisierbar ist und besonders gut für eine Array-Anordnung in der Röntgenkamera geeignet ist. Vorzugsweise ist der Strahlungsdetektor für eine Detektion von Röntgenstrahlung in einem breiten Energiebereich von 1 keV bis 300 keV, insbesondere oberhalb von 50 keV ausgelegt.

Vorteilhafterweise stellen die erfindungsgemäß zum Aufbau der Röntgenkamera verwendeten Strahlungsdetektoren Strahlensensoren dar, welche die photoleitenden Eigenschaften von _{"}wide bandgap" Halbleitern nutzen. Unter Strahleneinfluss ändert sich das leitende Detektorvolumen. Physikalisch ist dabei keine fest definierte elektrische Sperrschicht wie bei herkömmlichen Halbleiterdetektoren notwendig. Dies ermöglicht einen neuartigen Detektionsmodus für die Dosimetrie. Die Detektion der Strahlung beruht auf dem Prinzip eines Photoleiters mit internen Verstärkungseigenschaften. Es ist keine elektrische Sperrschicht erforderlich, sondern eine Volumenunabhängige Messung über die ohmschen Kontakte vorgesehen. Das Messsignal kann in verschiedener Weise (z. B. elektronisch, graphisch, akustisch) dargestellt werden.

Das Funktionsprinzip der erfindungsgemäß verwendeten GaN-Strahlungsdetektoren unterscheidet sich fundamental von dem der konventionell erhältlichen Halbleiterdetektoren für Röntgenstrahlung, bei denen Photo-induzierte Ladungsträger über ein elektrisches Feld gesammelt werden. Dagegen findet bei den GaN-Sensoren eine durch Strahlung hervorgerufene Änderung des Widerstandes statt (Photokonduktor), indem das Detektorvolumen verändert wird, in dem der Ladungstransport stattfindet. Obwohl auch in den GaN-Sensoren Raumladungszonen auftreten (in erster Linie durch Oberflächeneffekte), passiert der elektrische Strom parallel zu diesen Raumladungszonen den Halbleiter. Die Bestrahlung führt zu einem Nicht-Gleichgewicht von freien Landungsträgerkonzentrationen, die das Gesamtvolumen der Raumladungszonen und somit das Volumen verändern, welches zum Ladungstransport beiträgt. Daraus ergibt sich, dass die Höhe des Messsignals nicht direkt auf die Erzeugung von freien Ladungsträgern beschränkt ist, sondern eine massive interne Verstärkung stattfinden kann, wodurch im Vergleich zu herkömmlichen Techniken erhöhte Detektionssensitivitäten möglich sind.

Gemäß einem zweiten allgemeinen Gesichtspunkt der Erfindung wird ein Verfahren zur Aufnahme eines Bildes eines Untersuchungsgegenstandes unter Verwendung von Röntgenstrahlung bereitgestellt, bei dem die Röntgenkamera gemäß dem ersten Gesichtspunkt der Erfindung verwendet wird. Gemäß der Erfindung umfasst das Verfahren einen ersten Schritt der Bestrahlung des Untersuchungsgegenstands entlang mindestens einer vorbestimmten Bestrahlungsrichtung. Der Untersuchungsgegenstand wird im Strahlungsfeld einer Röntgenquelle positioniert. Gemäß der Erfindung umfasst das Verfahren des Weiteren einen zweiten Schritt der Detektion der Röntgenstrahlung nach einer Wechselwirkung mit dem Untersuchungsgegenstand. Dabei werden mit den Strahlungsdetektoren der erfindungsgemäßen Röntgenkamera ortsaufgelöst Dosiswerte erfasst, welche die örtliche Verteilung der Dosis im Strahlungsfeld der Röntgenstrahlung repräsentieren. Schließlich wird erfindungsgemäß in einem dritten Schritt mindestens ein Bild des Untersuchungsgegenstandes aus den ermittelten Dosiswerten rekonstruiert.

Gemäß einer bevorzugten Ausführungsform der Erfindung umfasst die Halteeinrichtung eine Halteplatte mit einer festen, vorbestimmten geometrischen Form. Es ist vorzugsweise eine Halteplatte aus einem starren Material, wie z.B. Kunststoff oder Keramik, vorgesehen. Vorzugsweise ist die Halteeinrichtung für eine Positionierung der Strahlungsdetektoren entlang einer geraden Bezugslinie oder einer ebenen Bezugsfläche ausgelegt. Vorteilhafterweise wird damit die Rekonstruktion eines Bildes aus den Messsignalen der Strahlungsdetektoren vereinfacht. Bei dieser Variante der Erfindung ist die Halteplatte vorzugsweise eben gebildet. Alternativ kann die Röntgenkamera in Abhängigkeit von der konkreten Anwendung an eine bestimmte geometrische Form des Untersuchungsgegenstandes angepasst sein. Die Halteeinrichtung kann konfiguriert sein, dass die Strahlungsdetektoren entlang einer gekrümmten Bezugslinie oder Bezugsfläche angeordnet sind. Bei dieser Variante der Erfindung umfasst die Halteeinrichtung vorzugsweise eine gekrümmte Halteplatte.

Vorteilhafterweise kann ein kompakter Aufbau der Röntgenkamera erzielt werden, wenn die Trägersubstrate der Strahlungsdetektoren unmittelbar mit der Halteeinrichtung fest verbunden, insbesondere auf der Halteplatte fixiert sind. Besonders bevorzugt ist eine Variante, bei der die Trägersubstrate und die Halteeinrichtung ein einstückiges Bauteil bilden. Die Trägersubstrate der Strahlungsdetektoren sind bei dieser Variante der Erfindung zu einem gemeinsamen Bauelement, insbesondere zu einer gemeinsamen Halteplatte verbunden. Beispielsweise kann ein Wafer, z.B. aus Silizium oder aus Saphir, gleichzeitig die Halteplatte und die Trägersubstrate der Strahlungsdetektoren bilden.

Gemäß einer weiteren bevorzugten Ausführungsform der Erfindung ist die Röntgenkamera mit einem Gehäuse versehen, das zur Aufnahme der Halteeinrichtung mit den Strahlungsdetektoren eingerichtet ist. Im Gehäuse sind vorzugsweise zusätzlich zur Halteeinrichtung mit den Strahlungsdetektoren auch Verbindungsleitungen zur Verbindung der Kontaktelektroden der Strahlungsdetektoren mit einer Messwertaufnehmereinrichtung aufgenommen.

Vorteilhafterweise bestehen verschiedene Möglichkeiten, Verbindungsleitungen zur elektrischen Verbindung der Kontaktelektroden der Strahlungsdetektoren mit der Messwertaufnehmereinrichtung bereitzustellen. Beispielsweise können für jeden Strahlungsdetektor jeweils zwei Verbindungsleitungen vorgesehen sein, welche die Kontaktelektroden mit der Messwertaufnehmereinrichtung verbinden. Diese Variante der Erfindung hat zwar einen komplexen Aufbau, da für eine bestimmte Anzahl von Strahlungsdetektoren die doppelte Anzahl von Verbindungsleitungen und eine entsprechende Schnittstelle an der Messwertaufnehmereinrichtung vorgesehen sein müssen. Vorteilhafterweise können jedoch an allen Strahlungsdetektoren gleichzeitig Messsignale (Detektorströme) erfasst werden. Alternativ können die Strahlungsdetektoren gruppenweise mit Verbindungsleitungen verbunden werden, um Messsignale der Strahlungsdetektoren seriell zu erfassen. Beispielsweise kann eine Matrixanordnung von Strahlungsdetektoren in geraden Reihen und Spalten vorgesehen sein. In diesem Fall kann jeder Reihe von Strahlungsdetektoren jeweils eine Verbindungsleitung und jeder Spalte von Strahlungsdetektoren jeweils eine Verbindungsleitung zugeordnet werden, wobei das Messsignal eines einzelnen Strahlungsdetektors erfasst werden kann, indem an die Verbindungsleitungen der zugehörigen Reihe und Spalte eine Messspannung angelegt wird. Vorteilhafterweise wird damit der Aufbau der Röntgenkamera vereinfacht, da eine erheblich verringerte Anzahl von Verbindungsleitungen erforderlich ist.

Besonders bevorzugt ist das Gehäuse flüssigkeitsdicht, resistent gegen Säuren, resistent gegen Basen, temperaturbeständig und/oder druckbeständig. Dies ermöglicht vorteilhafterweise die Anwendung der Röntgenkamera in extremen Umgebungsbedingungen, zum Beispiel zur abbildenden Dosimetrie in einem chemischen Reaktor.

Gemäß einer weiteren Variante kann die Röntgenkamera mit mindestens einer Haftoberfläche ausgestattet sein. Die Bereitstellung der Haftoberfläche bedeutet, dass die Röntgenkamera und insbesondere deren Gehäuse auf mindestens einer Oberfläche mit einer inhärenten Haftfähigkeit gebildet ist. Die Haftoberfläche weist zum Beispiel ein Klebemittel auf, das ein Anhaften der Röntgenkamera an einem Gegenstand, zum Beispiel auf der Oberfläche eines Untersuchungsgegenstands oder eines Probanden, erlaubt.

Ein weiterer Vorteil der Erfindung ist es, dass Röntgenstrahlung durch das Material der Halteeinrichtung und ggf. des Gehäuses der Röntgenkamera nur vernachlässigbar geschwächt wird. Daher können die sensitiven Elemente der Strahlungsdetektoren, d.h. die Detektorschichten durch die Trägersubstrate und optional die Halteeinrichtung und/oder das Gehäuse hindurch bestrahlt werden. Die Seite der Röntgenkamera, zu der die Trägersubstrate der Strahlungsdetektoren weisen, wird auch als Vorderseite oder Strahlungseintrittsfenster der Röntgenkamera bezeichnet, während die entgegengesetzte Seite, auf der sich die Kontaktelektroden und Verbindungsleitungen der Strahlungsdetektoren befinden, als Rückseite der Röntgenkamera bezeichnet wird. Vorteilhafterweise wird die Detektion der Röntgenstrahlung durch die Trägersubstrate hindurch, d. h. bei Bestrahlung der Röntgenkamera von der Vorderseite her, nicht durch die Kontaktelektroden oder die Verbindungsleitungen beeinträchtigt.

Gemäß einer weiteren bevorzugten Ausführungsform der Erfindung ist die Dicke der Detektorschichten geringer als 10 µm, insbesondere geringer als 5 µm. Vorzugsweise ist die Dicke der Detektorschichten größer als 100 nm, insbesondere größer als 500 nm. Die geringe Dicke der GaN-basierten Detektorschichten bietet Vorteile sowohl in Bezug auf die Herstellung der Strahlungsdetektoren als auch in Bezug auf deren Integration in der Röntgenkamera. Gemäß weiteren bevorzugten Ausführungsformen der Erfindung haben die Detektorschichten der einzelnen Pixel eine Fläche, die geringer als 100 mm², insbesondere geringer als 10 mm² ist. Die Fläche ist vorzugsweise größer als 1 µm², insbesondere größer als 0.1 mm². Die erfindungsgemäß verwendeten Strahlungsdetektoren sind durch lithographische Prozesse herstellbar und daher einfach miniaturisierbar. Die Detektorgröße (insbesondere Ausdehnung der empfindlichen Fläche) ist nahezu uneingeschränkt, insbesondere im Bereich von mehreren hundert µm bis zu 1 µm, bevorzugt in der Größe von 30 µm, skalierbar.

Vorzugsweise umfasst jeder Strahlungsdetektor eine einzige Detektorschicht. Mit anderen Worten, es ist ausschließlich eine Schicht vorgesehen, die mit ohmschen Kontakten ausgestattet und zur Erzeugung des Messsignals vorgesehen ist. Besonders bevorzugt besteht die Detektorschicht aus GaN, das optional eine Dotierung, zum Beispiel aus Eisen oder Kohlstoff, enthalten kann.

Weitere Vorteile für die Miniaturisierung des Strahlungsdetektors ergeben sich, wenn die Kontaktelektroden aus zwei Kontaktelektroden bestehen, die einseitig, das heißt insbesondere auf der zum Trägersubstrat entgegengesetzten Seite der Detektorschicht angeordnet sind. Die Kontakte befinden sich gemeinsam auf der selben Oberfläche der Detektorschicht.

Gemäß einer weiteren Variante kann die Messwertaufnehmereinrichtung Teil der Röntgenkamera sein. Die Röntgenkamera kann mit der Messwertaufnehmereinrichtung ausgestattet sein, die zum Beispiel mit der Halteeinrichtung verbunden oder optional in dem Gehäuse eingeschlossen oder über ein Kabel mit den Strahlungsdetektoren verbunden ist. Die Messwertaufnehmereinrichtung kann zur Strommessung und Spannungsversorgung, Datenspeicherung und/oder Datenübertragung eingerichtet sein. Die Strommessung bedeutet, dass mit der Messwertaufnehmereinrichtung elektrische Messsignale erfassbar sind, die für die in Reaktion auf Röntgenstrahlung in den Detektorschicht erzeugten Detektorströme charakteristisch sind. Wenn die Messwertaufnehmereinrichtung gemäß einer besonders bevorzugten Variante der Erfindung zur drahtlosen Kommunikation mit einer externen Steuereinrichtung konfiguriert ist und hierzu insbesondere eine RFID-Einrichtung umfasst, ergeben sich Vorteile für die Anwendung der Röntgenkamera in komplex aufgebauten Untersuchungsgegenständen.

Das örtliche Auflösungsvermögen der erfindungsgemäßen Röntgenkamera wird allgemein durch die Größe der Strahlungsdetektoren bestimmt. Um das Auflösungsvermögen bei einer gegebenen Größe der Strahlungsdetektoren zu verbessern, kann die Röntgenkamera gemäß einer weiteren vorteilhaften Ausführungsform der Erfindung mit einer Antriebseinrichtung ausgestattet sein, mit der die Röntgenkamera relativ zu dem Untersuchungsgegenstand beweglich ist. Die Antriebseinrichtung ist für eine Bewegung der Anordnung von Strahlungsdetektoren in einer von der Richtung der Röntgenstrahlung abweichenden Richtung konfiguriert. Vorzugsweise ist die Röntgenkamera mit der Antriebseinrichtung senkrecht zur Richtung der Röntgenstrahlung verschiebbar. Durch eine wiederholte Bildaufnahme mit einer jeweils relativ zum Untersuchungsgegenstand verschobenen Röntgenkamera können mehrere Bilder erfasst werden, deren Rekonstruktion ein Bild mit einer örtlichen Auflösung ergibt, die besser als die Größe der Strahlungsdetektoren ist.

Bevorzugte Anwendungen des erfindungsgemäßen Verfahrens sind beispielsweise bei der Computer-Tomographie (CT), bei der zweidimensionalen Röntgen-Bildgebung, bei der Überwachung einer Strahlentherapie und/oder bei einer Material- oder Gepäckprüfung gegeben. Für die CT-Anwendung der Erfindung wird die Detektion der Röntgenstrahlung entlang einer Zahl von Bestrahlungsrichtungen, die um mindestens 180° um den Untersuchungsgegenstand verteilt sind, wiederholt, um ein tomographisches Bild des Gegenstands rekonstruieren zu können. Die Rekonstruktion erfolgt dabei mit Rekonstruktionsverfahren, die an sich aus dem Stand der Technik bekannt sind.

Des Weiteren haben die erfindungsgemäß verwendeten Strahlungsdetektoren einen sehr großen Energiemessbereich, einen sehr großer Dosisratenmessbereich und nahezu keine Energieabhängigkeit im diagnostischen Röntgenbereich. Durch die extrem breiten Detektionsbereiche der GaN-Sensoren (Energie und Dosisrate), der Möglichkeit zur Biokompatibilisierung und die Möglichkeit zur Miniaturisierung, bieten die GaN-Sensoren ein erhebliches Potential im Bereich der Medizin sowie in medizin-technischen Anwendungen. Schließlich ermöglicht der Strahlungsdetektor eine 3-dimensionale Dosimetrie mit der Möglichkeit der Bildgebung.

Weitere Einzelheiten und Vorteile der Erfindung werden im Folgendem unter Bezug auf die in den beigefügten Zeichnungen dargestellten bevorzugten Ausführungsformen der Erfindung erläutert. Es zeigen in:
- Figuren 1A und 1B:: eine schematische Draufsicht auf die Strahlungsdetektoren einer erfindungsgemäßen Röntgenkamera und eine schematische Schnittansicht einer erfindungsgemäßen Röntgenkamera gemäß einer ersten Ausführungsform der Erfindung;
- Figur 2:: eine schematische Draufsicht auf die Strahlungsdetektoren und Verbindungsleitungen der erfindungsgemäßen Röntgenkamera gemäß einer weiteren Ausführungsform der Erfindung;
- Figuren 3A und 3B:: eine schematische Schnittansicht und eine schematische Draufsicht auf eine bevorzugte Ausführungsform eines Strahlungsdetektors einer erfindungsgemäßen Röntgenkamera;
- Figuren 4 und 5:: schematische Perspektivansichten verschiedener Varianten erfindungsgemäßer Röntgenkameras;
- Figur 6:: eine schematische Illustrationen der Positionierung einer erfindungsgemäßen Röntgenkamera am Körper eines Probanden; und
- Figur 7:: eine schematische Illustration einer erfindungsgemäßen Röntgenkamera gemäß einer weiteren Ausführungsform der Erfindung.

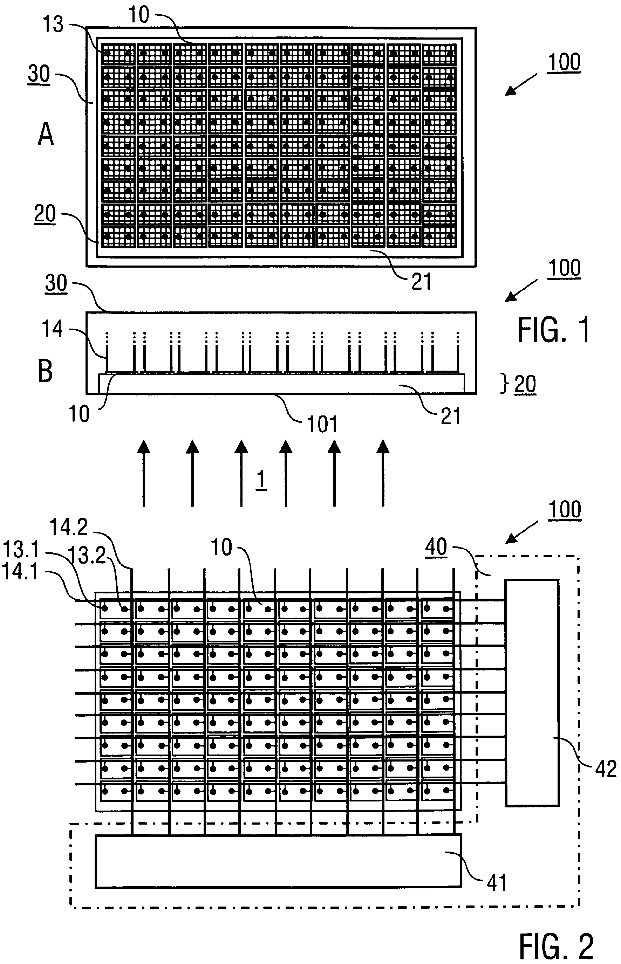

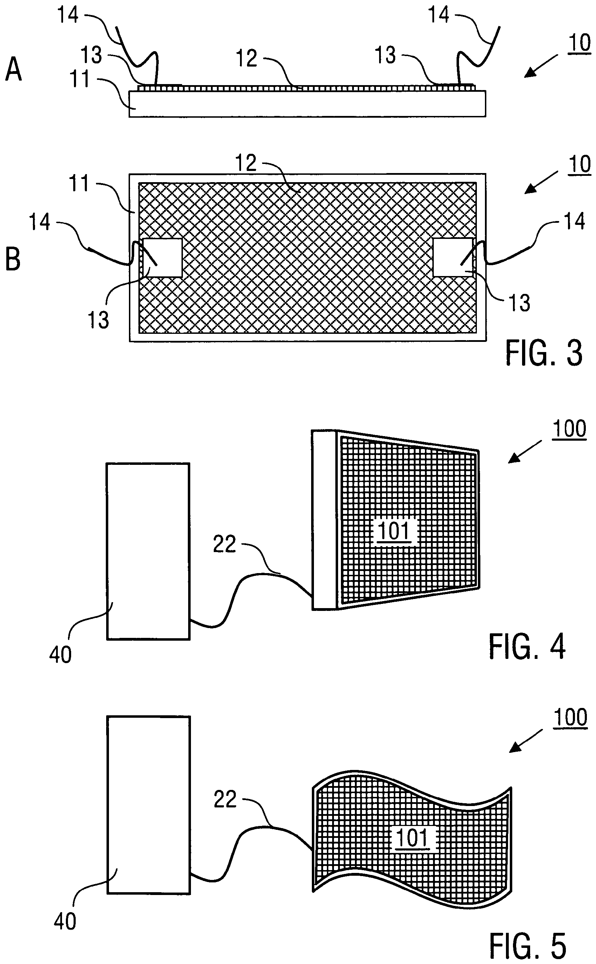

Die Figuren 1A und 1B illustrieren schematisch eine erfindungsgemäße Röntgenkamera 100 in schematischer Draufsicht auf die Anordnung der Strahlungsdetektoren (Figur 1A) und in schematischer Schnittansicht (Figur 1B) gemäß einer ersten Ausführungsform der Erfindung. Die Röntgenkamera 100 umfasst eine Vielzahl von Strahlungsdetektoren 10, die im dargestellten Beispiel matrixförmig in geraden Reihen und Spalten angeordnet sind. Alle Strahlungsdetektoren 10 der Röntgenkamera 100 sind gleich aufgebaut. Einzelheiten des Aufbaus der Strahlungsdetektoren 10, die jeweils ein Trägersubstrat, eine Detektorschicht und Kontaktelektroden umfassen, sind unten unter Bezug auf Figur 3 beschrieben. In einer abgewandelten Variante der Erfindung können die Strahlungsdetektoren mit einer anderen geometrischen Verteilung, z. B. in Kreisen oder als einzelne Reihe, und/oder mit verschiedenen Formen oder Größen vorgesehen sein. In einem praktischen Beispiel sind die Strahlungsdetektoren 10 auf einem 2-Zoll-Wafer angeordnet, der eine Fläche von ca. 2000 mm² aufweist. Es sind auch Kameras mit einer größeren sensitiven Fläche möglich, z. B. indem mehrere Wafer zusammengesetzt werden.

Die Strahlungsdetektoren 10 sind auf einer Halteeinrichtung 20 angeordnet, die eine Halteplatte 21 umfasst. Es ist eine ebene Halteplatte 21 aus einem Kunststoffmaterial, z.B. aus Polydimethylsiloxan (PDMS), PEN (Polyethylennaphthalat) oder Polyethylenterephthalat (PET) vorgesehen. Die Strahlungsdetektoren 10 sind auf der Halteplatte 21 aufgeklebt. Die ebene Halteplatte 21 bildet eine ebene Bezugsfläche, entlang derer die Strahlungsdetektoren 10 angeordnet sind.

Gemäß einer abgewandelten Variante der Erfindung sind die Trägersubstrate der Strahlungsdetektoren 10 durch ein gemeinsames Substrat, z. B. aus Saphir, gebildet, das gleichzeitig die Halteplatte der Halteeinrichtung 20 ist. Mit anderen Worten, die Bereitstellung der Halteplatte ist nicht zwingend erforderlich. Zum Beispiel das Saphirsubstrat, auf dem die Strahlungsdetektoren 10 gebildet sind, ist ausreichend stabil und kann daher selbst als Halteplatte dienen.

Auf der frei liegenden Oberfläche der Strahlungsdetektoren 10 sind Verbindungsleitungen 14 vorgesehen, welche die Kontaktelektroden 13 der Strahlungsdetektoren 10 mit der Messwertaufnehmereinrichtung (nicht dargestellt) zur Messwertaufnahme und zur Steuerung der Röntgenkamera 100 verbinden. Beim dargestellten Beispiel sind an jedem Strahlungsdetektor 10 zwei Verbindungsleitungen 14 vorgesehen, die mit der elektronischen Schaltung verbunden sind. Die von den Verbindungsleitungen 14 wegweisende Seite der Anordnung der Strahlungsdetektoren 10 bildet die Vorderseite oder das Strahlungseintrittsfenster 101 der Röntgenkamera 100, während die entgegengesetzte Seite, auf der sich die Verbindungsleitungen 14 befinden, die Rückseite der Röntgenkamera 100 bildet.

Zur ortsaufgelösten Detektion von Röntgenstrahlung 1 wird die Röntgenkamera 100 so positioniert, dass die Röntgenstrahlung 1 auf das Strahlungseintrittsfenster 101 gerichtet ist. Die an den einzelnen Strahlungsdetektoren 10 gemessenen Dosiswerte werden erfasst, indem an jeden Strahlungsdetektor 10 eine Messspannung angelegt und der am Strahlungsdetektor 10 fließende Detektorstrom gemessen wird. Der Detektorstrom hängt von der Dosis der einfallenden Röntgenstrahlung 1 ab. Die gemessenen Detektorströme liefern Dosiswerte, aus denen eine durch die Röntgenstrahlung 1 repräsentierte Bildinformation rekonstruiert werden kann.

Die Röntgenkamera 100 ist gemäß Figur 1 mit einem Gehäuse 30 ausgestattet, das die Strahlungsdetektoren 10 und die Halteeinrichtung 20 sowie die Verbindungsleitungen 14 einschließt. Auf der Vorderseite 101 der Röntgenkamera 100 kann das Gehäuse 30 durch die Halteplatte 21 gebildet werden, d.h. an der Vorderseite 101 kann die Halteplatte 21 frei liegen.

Figur 2 illustriert schematisch eine abgewandelte Ausführungsform der Erfindung, bei der abweichend von Figur 1 nicht jeder Strahlungsdetektor 10 mit zwei Verbindungsleitungen 14, sondern Gruppen von Strahlungsdetektoren 10 mit Verbindungsleitungen 14.1, 14.2 verbunden sind. Die Strahlungsdetektoren 10 sind matrixförmig mit geraden Reihen und Spalten angeordnet. Jeder Strahlungsdetektor 10 hat eine erste Kontaktelektrode 13.1 und eine zweite Kontaktelektrode 13.2. Die ersten Kontaktelektroden 13.1 sind reihenweise mit einer gemeinsamen Verbindungsleitung 14.1 verbunden, während die zweiten Kontaktelektroden 13.2 spaltenweise mit einer gemeinsamen Verbindungsleitung 14.2 verbunden sind. Die Zahl der ersten Verbindungsleitungen 14.1 ist somit gleich der Zahl der Reihen der Matrix-Anordnung der Strahlungsdetektoren 10, während die Zahl der zweiten Verbindungsleitungen 14.2 gleich der Zahl der Spalten der Matrix-Anordnung der Strahlungsdetektoren 10 ist. Die ersten und zweiten Verbindungsleitungen 14.1, 14.2 sind mit Multiplex-Schaltungen 41, 42 der Messwertaufnehmereinrichtung 40 verbunden. Die Multiplex-Schaltungen 41, 42 sind dazu konfiguriert, aufeinander folgend Strahlungsdetektoren 10 mit Messspannungen zu beaufschlagen, um an den einzelnen Strahlungsdetektoren 10 Detektorströme zur ortsaufgelösten Dosismessung zu erfassen.

Die Strahlungsdetektoren 10 der erfindungsgemäßen Röntgenkamera 100 sind z.B. aufgebaut, wie dies im Folgenden unter Bezug auf die Figuren 3A und 3B erläutert ist.

Gemäß den Figuren 3A und 3B umfasst jeder Strahlungsdetektor 10 ein Trägersubstrat 11, eine Detektorschicht 12 und Kontaktelektroden 13, die über die Verbindungsleitungen 14 mit der Messwertaufnehmereinrichtung zur Strommessung (nicht dargestellt) verbunden werden können. Das Trägersubstrat 11 umfasst zum Beispiel Saphir mit einer Dicke von 0,33 mm. Die Detektorschicht 12 besteht aus GaN mit einer Dicke von zum Beispiel 2,5 µm. Die Kontaktelektroden 13 bestehen zum Beispiel aus Ti/Al. Sie sind mittels thermischem Aufdampfen oder Elektronenstrahl-Aufdampfen auf der GaN-Detektorschicht 12 fixiert, so dass zwischen den Kontaktelektroden 13 und der Detektorschicht 12 jeweils ein ohmscher Kontakt gebildet wird. Die Maße der Detektorschicht 12 (Figur 1B) betragen zum Beispiel 0,5 mm · 2 mm, während die Maße der Kontaktelektroden 13 zum Beispiel jeweils 300 µm · 300 µm betragen. Der Strahlungsdetektor 10 ist für Röntgenstrahlung mit einer Energie von 20 keV bis 300 keV bis in den µGy-Bereich empfindlich. Wie oben erwähnt, muss nicht jeder Detektor ein separates Trägersubstrat haben. Es ist gemäß der Erfindung möglich, die einzelnen Detektorschichten (Pixel) aus einer einzigen, zusammenhängenden Detektorschicht (Wafer), zum Beispiel durch Ätzen, auf einem gemeinsamen Substrat zu bilden. Das Substrat wird dabei zwischen den Pixeln nicht getrennt. Die Figuren 4 und 5 illustrieren schematisch zwei Varianten der erfindungsgemäßen Röntgenkamera 100 mit einer ebenen (Figur 4) oder einer gekrümmten (Figur 5) Form des Strahlungseintrittsfensters 101. Gemäß Figur 4 sind die Strahlungsdetektoren im Gehäuse 20 untergebracht, von dem ein Verbindungskabel 22 zu der Messwertaufnehmereinrichtung 40 verläuft, in der die Messwertaufnahme und -speicherung und ggf. die Rekonstruktion des erfassten Bildes erfolgen. Gemäß Figur 5 ist kein Gehäuse vorgesehen. In diesem Fall sind die Strahlungsdetektoren 10 mit den Verbindungsleitungen auf der Rückseite der Röntgenkamera 100 mit einer Kunststoffschicht verkapselt. Wiederum führt ein Verbindungskabel 22 zu der Messwertaufnehmereinrichtung 40, in der die Messwertaufnahme und -speicherung sowie optional die Bildrekonstruktion vorgesehen sind.

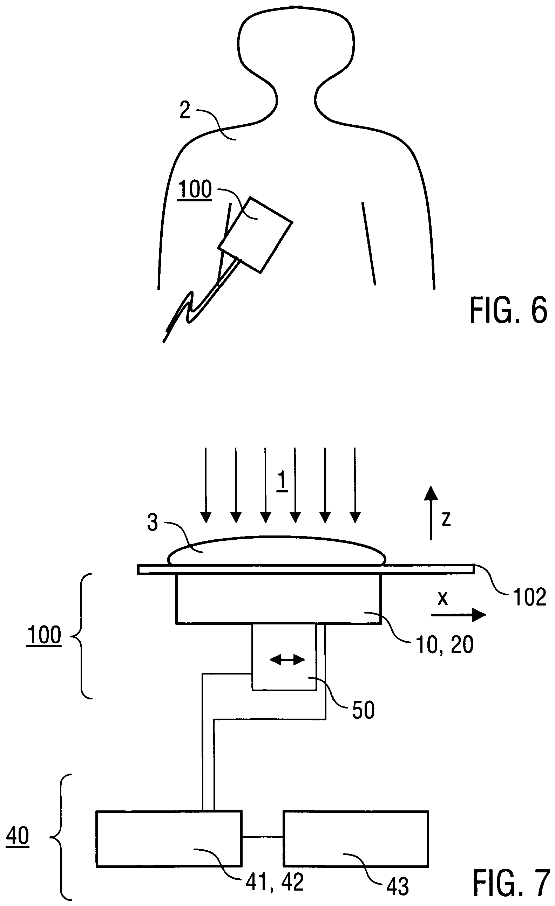

Figur 6 veranschaulicht schematisch die Positionierung von Röntgenkamera 100 gemäß der Erfindung auf der Hautoberfläche eines Probanden 2. Wenn das Gehäuse der Röntgenkamera 100 eine Haftoberfläche aufweist, kann die Röntgenkamera 100 ohne weitere Hilfsmittel direkt auf der Hautoberfläche fixiert werden. Alternativ ist eine subkutane Anordnung oder eine Anordnung in einem Hohlraum des Probandenkörpers möglich.

Figur 7 illustriert schematisch eine weitere Ausführungsform einer erfindungsgemäßen Röntgenkamera 100, die zum Beispiel für die bildgebende Dosimetrie bei der Materialprüfung eingerichtet ist. Die Röntgenkamera 100 ist mit einer Antriebseinrichtung 50 verbunden, mit der die Röntgenkamera 100 relativ zu einem Tisch 102 verschiebbar ist. Der Tisch 102 ist zur Aufnahme des Untersuchungsgegenstandes 3 vorgesehen. Bei Bestrahlung des Untersuchungsgegenstands 3 mit Röntgenstrahlung 1 wird das Strahlungsfeld mit der Röntgenkamera 100 ortsaufgelöst erfasst. Die Antriebseinrichtung 50 und die Strahlungsdetektoren 10 der Röntgenkamera 100 sind mit der Messwertaufnehmereinrichtung 40 verbunden, die für die Messwertaufnahme und -speicherung und auch für die Steuerung der Komponenten 10, 50 vorgesehen ist. Die Bildrekonstruktion kann in einer gesonderten Rekonstruktionsschaltung 43 erfolgen, die mit der Messwertaufnehmereinrichtung 40 verbunden ist.

Zur Verbesserung der örtlichen Auflösung der Bildaufnahme kann mit der Antriebseinrichtung 50 die Röntgenkamera 100 in einer Versatzrichtung (x-Richtung) senkrecht zur Bestrahlungsrichtung (z-Richtung) verfahren werden, um aufeinander folgend mehrere Bilder des durchstrahlten Untersuchungsgegenstands 3 aufzunehmen. Für jedes Bild wird die Röntgenkamera relativ zum Untersuchungsgegenstand in der Versatzrichtung verschoben. Der Betrag der Verschiebung ist kleiner als die Ausdehnung Δx der Strahlungsdetektoren in der Versatzrichtung. Wenn der Betrag der Verschiebung in der Versatzrichtung ein ganzzahliger Teil der Ausdehnung Δx/N des Strahlungsdetektors ist, sind vorzugsweise entsprechend N Bildaufnahmen jeweils mit verschiedenen Positionen der Röntgenkamera relativ zum Untersuchungsgegenstand 3 vorgesehen. Aus den N Bildaufnahmen kann ein Bild mit einer im Vergleich zu einem Einzelbild verbesserten örtlichen Auflösung rekonstruiert werden.

Die in der vorstehenden Beschreibung, den Zeichnungen und den Ansprüchen offenbarten Merkmale der Erfindung können sowohl einzeln als auch in Kombination für die Verwirklichung der Erfindung in ihren verschiedenen Ausgestaltungen von Bedeutung sein.

## Patentansprüche

1. Röntgenkamera (100) zur ortsaufgelösten Detektion von Röntgenstrahlung (1), umfassend:
- eine Vielzahl von Strahlungsdetektoren (10), die jeweils ein Trägersubstrat (11), eine einzige Detektorschicht (12), Kontaktelektroden (13) und Verbindungsleitungen (14) aufweisen, wobei die Detektorschicht (12) GaN enthält und auf dem Trägersubstrat (11) angeordnet ist, die Kontaktelektroden (13) mit der Detektorschicht (12) ohmsche Kontakte bilden, und die Verbindungsleitungen (14) angeordnet sind, die Kontaktelektroden (13) mit einer Messwertaufnehmereinrichtung zu verbinden,
**dadurch gekennzeichnet, dass**
- die Detektorschichten (12) der Strahlungsdetektoren (10) jeweils eine Dicke geringer als 50 µm aufweisen, und
- die Röntgenkamera (100) eine Halteeinrichtung (20) mit einer Halteplatte (21) mit einer vorbestimmten geometrischen Form umfasst, mit der die Strahlungsdetektoren (10) entlang einer vorbestimmten Bezugslinie oder Bezugsfläche verbunden sind, wobei
- die Strahlungsdetektoren (10) mit den Trägersubstraten (11) so an der Halteplatte (21) angeordnet sind, dass die Trägersubstrate (11) einheitlich zu einer gemeinsamen Vorderseite, die ein Strahlungseintrittsfenster (101) bildet, weisen und die Kontaktelektroden (13) einheitlich zu einer gemeinsamen Rückseite der Röntgenkamera (100) weisen, wobei sich die Verbindungsleitungen (14) an der Rückseite befinden.

2. Röntgenkamera gemäß Anspruch 1, bei der
- die Trägersubstrate (11) der Strahlungsdetektoren (10) mit der Halteeinrichtung (20) verbunden sind,
- die Trägersubstrate (11) der Strahlungsdetektoren (10) durch ein gemeinsames Substrat gebildet werden,
- die Trägersubstrate (11) der Strahlungsdetektoren (10) die Halteeinrichtung (20) bilden, und/oder
- die Trägersubstrate (11) der Strahlungsdetektoren (10) und die Halteeinrichtung (20) ein einstückiges Bauteil bilden.

3. Röntgenkamera gemäß einem der vorhergehenden Ansprüche, bei der
- die Bezugslinie oder Bezugsfläche (21) gerade, eben oder gekrümmt ist.

4. Röntgenkamera gemäß einem der vorhergehenden Ansprüche, bei der
- ein Gehäuse (30) zur Aufnahme der Halteeinrichtung (20) mit den Strahlungsdetektoren (10) vorgesehen ist.

5. Röntgenkamera gemäß Anspruch 4, bei der
- das Gehäuse (30) flüssigkeitsdicht, resistent gegen Säuren, resistent gegen Basen, temperaturbeständig und/oder druckbeständig ist, und/oder
- das Gehäuse (30) mit einer Haftoberfläche ausgestattet ist.

6. Röntgenkamera gemäß einem der vorhergehenden Ansprüche, bei der
- die Strahlungsdetektoren (10) matrixförmig in geraden Reihen und Spalten angeordnet sind.

7. Röntgenkamera gemäß einem der vorhergehenden Ansprüche, bei der
- die Dicke der Detektorschichten (12) geringer als 10 µm, insbesondere geringer als 5 µm ist,
- die Detektorschichten (12) eine Fläche aufweisen, die geringer als 100 mm², insbesondere geringer als 10 mm² ist,
- die Kontaktelektroden zwei Kontaktelektroden (13) umfassen, die einseitig auf der Detektorschicht (12) angeordnet sind, und/oder
- das GaN mit Fe oder C dotiert ist.

8. Röntgenkamera gemäß einem der vorhergehenden Ansprüche, die
- eine Messwertaufnehmereinrichtung (40), insbesondere mit einer RFID-Einrichtung, aufweist, die zur Strommessung, Datenspeicherung und/oder -übertragung eingerichtet ist.

9. Röntgenkamera gemäß einem der vorhergehenden Ansprüche, die
- eine Antriebseinrichtung (50) aufweist, die zur Bewegung der Strahlungsdetektoren (10) relativ zu einem zu untersuchenden Gegenstand (3) konfiguriert ist.

10. Verfahren zur Aufnahme eines Bildes eines Untersuchungsgegenstandes (2, 3) unter Verwendung von Röntgenstrahlung (1), mit den Schritten:
- Bestrahlung des Untersuchungsgegenstandes (2, 3) entlang mindestens einer vorbestimmten Bestrahlungsrichtung (z),
- Detektion der Röntgenstrahlung (1) nach einer Wechselwirkung mit dem Untersuchungsgegenstand (2, 3), wobei eine Röntgenkamera (100) gemäß einem der vorhergehenden Ansprüche verwendet wird und mit den Strahlungsdetektoren (10) der Röntgenkamera (100) ortsaufgelöst Dosiswerte erfasst werden, und
- Rekonstruktion mindestens eines Bildes des Untersuchungsgegenstandes (2, 3) aus den erfassten Dosiswerten.

11. Verfahren gemäß Anspruch 10, bei dem
- die Bestrahlung des Untersuchungsgegenstandes (2, 3) und die Detektion der Röntgenstrahlung (1) entlang einer Vielzahl von verschiedenen Bestrahlungsrichtungen wiederholt wird, und
- bei der Rekonstruktion ein tomographisches Bild des Untersuchungsgegenstandes (2, 3) ermittelt wird.

12. Verfahren gemäß Anspruch 10, bei dem
- die Detektion der Röntgenstrahlung (1) entlang der mindestens einen vorbestimmten Bestrahlungsrichtung (z) mehrfach wiederholt wird, wobei die Röntgenkamera (100) jeweils in einer von der Bestrahlungsrichtung abweichenden Versatzrichtung (x) verschoben wird und wobei der Betrag der Verschiebung in der Versatzrichtung (x) gleich einem Bruchteil der Ausdehnung der Strahlungsdetektoren (10) in der Versatzrichtung (x) ist.

13. Verwendung einer Röntgenkamera (100) gemäß einem der Ansprüche 1 bis 9 zur abbildenden Dosimetrie von Röntgenstrahlung, insbesondere zur Computer-Tomographie, 2D-Röntgen-Bildgebung, Überwachung einer Strahlentherapie oder Material- oder Gepäckprüfung.

## Claims

1. X-ray camera (100) for spatially resolved detection of X-ray radiation (1), comprising:
- a plurality of radiation detectors (10), each of which having a carrier substrate (11), one single detector layer (12), contact electrodes (13) and connection lines (14), wherein the detector layer (12) contains GaN and is arranged on the carrier substrate (11), the contact electrodes (13) form ohmic contacts with the detector layer (12), and the connection lines (14) are arranged for connecting the contact electrodes (13) with a measuring element device,
**characterized in that**
- the detector layers (12) of the radiation detectors (10) each have a thickness of less than 50 µm, and
- the X-ray camera (100) comprises a holding device (20) with a holding plate (21) having a predetermined geometric shape, the radiation detectors (10) being connected with the holding plate (21) along a predetermined reference line or reference surface, wherein
- the radiation detectors (10) with the carrier substrates (11) are arranged on the holding plate (21) such that the carrier substrates (11) are uniformly directed towards a common front side, which forms a radiation entry window (101), and the contact electrodes (13) are uniformly directed towards a common rear side of the X-ray camera (100), wherein the connecting lines (14) are located on the rear side.

2. X-ray camera according to claim 1, wherein
- the carrier substrates (11) of the radiation detectors (10) are connected with the holding device (20),
- the carrier substrates (11) of the radiation detectors (10) are formed by a common substrate,
- the carrier substrates (11) of the radiation detectors (10) form the holding device (20), and/or
- the carrier substrates (11) of the radiation detectors (10) and the holding device (20) form an integral component.

3. X-ray camera according to one of the preceding claims, wherein
- the reference line or reference surface (21) is straight, plane or curved.

4. X-ray camera according to one of the preceding claims, wherein
- a housing (30) is provided for accommodating the holding device (20) with the radiation detectors (10).

5. X-ray camera according to claim 4, wherein
- the housing (30) is liquid-tight, resistant against acids, resistant against bases, temperature-proof and/or pressure-resistant, and/or
- the housing (30) is provided with an adhesive surface.

6. X-ray camera according to one of the preceding claims, wherein
- the radiation detectors (10) are arranged matrix-shaped in straight rows and columns.

7. X-ray camera according to one of the preceding claims, wherein
- the thickness of the detector layers (12) is less than 10 µm, in particular less than 5 µm,
- the detector layers (12) have a surface, which is less than 100 mm², in particular less than 10 mm²,
- the contact electrodes comprise two contact electrodes (13), which are arranged on one side on the detector layer (12), and/or
- the GaN is doped with Fe or C.

8. X-ray camera according to one of the preceding claims, which
- has a measuring element device (40), in particular with an RFID device, which is adapted for current measurement, data storage and/or data transmission.

9. X-ray camera according to one of the preceding claims, which
- has a drive device (50), which is configured for movement of the radiation detectors (10) relative to an object (3) to be investigated.

10. Method for collecting an image of an object (2, 3) to be investigated using X-ray radiation (1), with the steps of:
- irradiation of the object (2, 3) to be investigated along at least one predetermined direction of irradiation (z),
- detection of the X-ray radiation (1) after an interaction with the object (2, 3) to be investigated, wherein an X-ray camera (100) according to one of the preceding claims is used and dosage values are recorded in a spatially resolved manner with the radiation detectors (10) of the X-ray camera (100), and
- reconstruction of at least one image of the object (2, 3) to be investigated from the recorded dosage values.

11. Method according to claim 10, wherein
- the irradiation of the object (2, 3) to be investigated and the detection of the X-ray radiation (1) is repeated along a plurality of different directions of irradiation, and
- with the reconstruction, a tomographic image of the object (2, 3) to be investigated is determined.

12. Method according to claim 10, wherein
- the detection of the X-ray radiation (1) along the at least one predetermined direction of irradiation (z) is repeated several times, wherein the X-ray camera (100) is shifted in respectively one offset direction (x), which deviates from the direction of irradiation, and wherein the amount of the displacement in the offset direction (x) is equal to a fraction of the extension of the radiation detectors (10) in the offset direction (x).

13. Use of an X-ray camera (100) according to one of the claims 1 to 9 for imaging dosimetry of X-ray radiation, in particular for computer tomography, 2-dimensional X-ray imaging, monitoring of a radiotherapy or material check or luggage check.

## Revendications

1. Caméra à rayonnement X (100) pour la détection à résolution spatiale de rayonnement X (1), comprenant :
- une pluralité de détecteurs de rayonnement (10) qui présentent respectivement un substrat porteur (11), une seule couche de détecteur (12), des électrodes de contact (13) et des câbles de raccordement (14), dans laquelle la couche de détecteur (12) contient du GaN et est disposée sur le substrat porteur (11), les électrodes de contact (13) forment des contacts ohmiques avec la couche de détecteur (12), et les câbles de raccordement (14) sont disposés afin de raccorder les électrodes de contact (13) à un dispositif transducteur,
**caractérisé en ce que**
- les couches de détecteur (12) des détecteurs de rayonnement (10) présentent respectivement une épaisseur inférieure à 50 µm, et
- la caméra à rayonnement X (100) comporte un dispositif de retenue (20) avec une plaque de retenue (21) possédant une forme géométrique prédéterminée, avec laquelle les détecteurs de rayonnement (10) sont raccordés le long d'une ligne de référence ou surface de référence prédéterminée, dans laquelle
- les détecteurs de rayonnement (10) sont disposés avec les substrats porteurs (11) sur la plaque de retenue (21) de sorte que les substrats porteurs (11) soient dirigés uniformément vers un côté avant commun qui forme une fenêtre d'entrée de rayonnement (101) et les électrodes de contact (13) soient dirigées uniformément vers un côté arrière commun de la caméra à rayonnement X (100), dans laquelle les câbles de raccordement (14) se trouvent sur le côté arrière.

2. Caméra à rayonnement X selon la revendication 1, où
- les substrats porteurs (11) des détecteurs de rayonnement (10) sont raccordés au dispositif de retenue (20),
- les substrats porteurs (11) des détecteurs de rayonnement (10) sont formés par un substrat commun,
- les substrats porteurs (11) des détecteurs de rayonnement (10) forment le dispositif de retenue (20), et/ou
- les substrats porteurs (11) des détecteurs de rayonnement (10) et le dispositif de retenue (20) forment un composant d'un seul tenant.

3. Caméra à rayonnement X selon l'une quelconque des revendications précédentes, où
- la ligne de référence ou surface de référence (21) est droite, plane ou courbée.

4. Caméra à rayonnement X selon l'une quelconque des revendications précédentes, où
- un boîtier (30) est prévu pour la réception du dispositif de retenue (20) avec les détecteurs de rayonnement (10).

5. Caméra à rayonnement X selon la revendication 4, où
- le boîtier (30) est étanche au liquide, résistant aux acides, résistant aux bases, résistant aux températures et/ou résistant à la pression, et/ou
- le boîtier (30) est équipé d'une surface adhérente.

6. Caméra à rayonnement X selon l'une quelconque des revendications précédentes, où
- les détecteurs de rayonnement (10) sont disposés en forme de matrice dans des rangées et colonnes droites.

7. Caméra à rayonnement X selon l'une quelconque des revendications précédentes, où
- l'épaisseur des couches de détecteur (12) est inférieure à 10 µm, en particulier inférieure à 5 µm,
- les couches de détecteur (12) présentent une surface qui est inférieure à 100 mm², en particulier inférieure à 10 mm²,
- les électrodes de contact comportent deux électrodes de contact (13) qui sont disposées d'un seul côté sur la couche de détecteur (12), et/ou
- le GaN est dopé avec du Fe ou C.

8. Caméra à rayonnement X selon l'une quelconque des revendications précédentes, qui
- présente un dispositif transducteur (40), en particulier avec un dispositif RFID qui est aménagé pour la mesure du courant, l'enregistrement et/ou la transmission de données.

9. Caméra à rayonnement X selon l'une quelconque des revendications précédentes, qui
- présente un dispositif d'entraînement (50) qui est configuré pour le mouvement des détecteurs de rayonnement (10) par rapport à un objet à examiner (3).

10. Procédé d'enregistrement d'une image d'un objet à examiner (2, 3) en utilisant un rayonnement de rayonnement X (1), avec les étapes suivantes :
- l'irradiation de l'objet à examiner (2, 3) le long d'au moins un sens d'irradiation (z) prédéterminé,
- la détection du rayonnement X (1) après une interaction avec l'objet à examiner (2, 3), dans lequel une caméra à rayonnement X (100) selon l'une quelconque des revendications précédentes est utilisée et des valeurs de dose sont détectées par résolution spatiale avec les détecteurs de rayonnement (10) de la caméra à rayonnement X (100), et
- la reconstruction d'au moins une image de l'objet à examiner (2, 3) à partir des valeurs de dose détectées.

11. Procédé selon la revendication 10, où
- l'irradiation de l'objet à examiner (2, 3) et la détection du rayonnement X (1) sont répétées le long d'une pluralité de différents sens d'irradiation, et
- une image tomographique de l'objet à examiner (2, 3) est déterminée lors de la reconstruction.

12. Procédé selon la revendication 10, où
- la détection du rayonnement X (1) est répétée le long d'au moins un sens d'irradiation (z) prédéterminé à plusieurs reprises, dans lequel la caméra à rayonnement X (100) est déplacée respectivement dans un sens de déport (x) divergeant du sens d'irradiation et dans lequel la valeur du déplacement dans le sens de déport (x) est identique à une fraction de l'extension des détecteurs de rayonnement (10) dans le sens de déport (x).

13. Utilisation d'une caméra à rayonnement X (100) selon l'une quelconque des revendications 1 à 9 pour la dosimétrie de représentation de rayonnement X, en particulier pour la tomodensimétrie, l'imagerie à rayonnement X en 2D, la surveillance d'une radiothérapie ou le contrôle des matériaux ou des bagages.
